# EUROPEAN PATENT SPECIFICATION

(11) **EP 3 969 103 B1**
(45) Date of publication and mention of the grant of the patent: **02.07.2025**
(21) Application number: 20726108.2
(22) Date of filing: 14.05.2020
(51) Int. Cl.: A61N 1/36, A61B 5/021, A61B 5/026

(54) **SYSTEM FOR CLOSED LOOP CONTROL OF AUTONOMIC FUNCTION**
SYSTEM ZUR STEUERUNG MIT GESCHLOSSENEM REGELKREIS EINER AUTONOMEN FUNKTION
SYSTÈME DE COMMANDE EN BOUCLE FERMÉE DE FONCTION AUTONOME

(30) Priority: 15.05.2019 EP 19174558; 15.05.2019 EP 19174566
(43) Date of publication of application: 23.03.2022
(73) Proprietor: ECOLE POLYTECHNIQUE FEDERALE DE LAUSANNE (EPFL), 1015 Lausanne (CH); UTI Limited Partnership, Calgary, Alberta T2L 2K7 (CA); Regents of the University of Minnesota, Minneapolis, MN 55455 (US)
(72) Inventor: COURTINE, Grégoire, 1005 Lausanne (CH); PHILLIPS, Aaron, Calgary, Alberta T2P 0X2 (CA); SQUAIR, Jordan, 1003 Lausanne (CH); DARROW, David, Minneapolis, MN 55407-5540 (US)
(74) Representative: DTS Patent- und Rechtsanwälte PartmbB
(86) International application number: PCT/EP2020/063563
(87) International publication number: WO 2020/229645

(56) References cited:
- WO-A1-2018/148844
- US-A1- 2007 156 200
- US-A1- 2011 202 107
- US-A1- 2013 289 650
- US-A1- 2017 079 598

## Description

The present invention refers to the field of spinal cord neuro-prostheses, in particular for rehabilitation of autonomic functions.

In particular, it refers to a system for stimulation of the spinal cord, more in particular for the rehabilitation of autonomic function, in particular blood pressure, in patients with spinal cord injury or other disorders (e.g. stroke, multiple sclerosis, autonomic failure, autonomic neuropathy or cancer of the neurological tissue which impair operation of descending sympathetic pathways that normally facilitate control of autonomic functions).

The spinal cord is an integral part of the central nervous system (CNS). Spinal cord injury (SCI) results in motor and sensory deficits but also in autonomic dysfunctions. SCI results in disconnection of some, most, or all descending sympathetic pathways that carry signals responsible for regulating arterial blood pressure, heart rate and/or gut and bladder function. Autonomic dysfunction following SCI is a potentially life-threatening condition that leads to blood pressure instability and ensuing chronic dysfunction of the heart and vasculature. Most SCI patients experience multiple drastic, blood pressure fluctuations daily, and rank this as a top healthcare priority.

Individuals with severe SCI may suffer from dysregulated cardiovascular control. In the acute phase after injury this manifests as severe resting hypotension, requiring intensive care unit physicians to rigorously monitor hemodynamics and to elevate blood pressure using pharmacology (e.g., norepinephrine, phenylephrine, dobutamine). However, these drugs are short acting, and can lead to large elevations in blood pressure, which then force the clinician to reduce the dose, often leading to an episode of very low blood pressure (i.e., hypoperfusion). In the chronic phase after injury (i.e., 6 months onward), individuals with these severe SCIs have dramatic bouts of orthostatic hypotension (a decrease in blood pressure of equal to or more than 20 mmHg systolic pressure). These episodes of hypotension occur on a daily-basis and lead to classic presyncopal symptoms and impair quality of life.

Severe SCI disconnects brainstem control centers from the sympathetic circuitry within the thoracic spinal cord that is responsible for blood pressure regulation. Loss of this regulation results in significant hypotension, both in the acute phase after injury (i.e., in the emergency room and intensive care unit immediately following the injury) and in the sub-acute/chronic phase (i.e., following discharge from the intensive care unit). In both cases, epidural electrical stimulation (EES) of the spinal cord can play a role in stabilizing blood pressure.

Blood pressure is the pressure of circulating blood on the walls of blood vessels. Without further specification, 'blood pressure' often may refer to the pressure in arteries of the systemic circulation. Blood pressure may usually be expressed in terms of the systolic pressure, i.e. the maximum pressure during one heartbeat and/or over diastolic pressure, i.e. the minimum pressure in between two heartbeat and/or over mean arterial blood pressure, i.e. an average blood pressure in an individual during a single cardiac cycle, and may be measured in millimeters of mercury (mmHg, above the surrounding atmospheric pressure).

Blood pressure monitoring may comprise monitoring a parameter value such as a diastolic blood pressure, a systolic blood pressure, a diastolic blood pressure and a systolic blood pressure, a mean arterial pressure, a blended blood pressure value or the like.

Further, perfusion pressure, i.e. spinal cord perfusion pressure, which is defined as the difference between mean arterial blood pressure and cerebrospinal fluid pressure may be of importance. The latter may be monitored using intrathecal catheters placed e.g. near the site of injury or in the lumbar cistern of the injured subject/patient.

When blood pressure, in particular arterial blood pressure, decreases or increases as a consequence of SCI, the spinal cord neurons responsible for blood pressure control no longer have the capacity to maintain blood pressure at a normal, physiological level. This disconnection of sympathetic control can lead to a situation where blood vessels do not maintain appropriate tone (e.g. the blood vessels can become dilated). Large amounts of blood may pool in subjects' lower part of the body, e.g. legs and gut. Consequently, subjects affected by SCI can suffer from extremely low blood pressure, i.e. hypotension. Individuals with SCI are often unable to regulate their blood pressure. These individuals typically experience very low arterial blood pressure at rest, during exercise and/or when assuming a seated or standing position. This hypotension may lead to dizziness, disorientation, reduction in cognitive functioning, loss of consciousness and a predisposition to strokes and heart attacks. Additionally, dangerous elevations in blood pressure, i.e. hypertension may also result from SCI. Hypertension may lead to heart attacks, strokes, and sub-clinical vascular consequences. As described above, autonomic cardiovascular dysfunctions following SCI are a top health priority. One primary autonomic issue after high-level SCI (i.e., above the 6th thoracic segment) is orthostatic hypotension, which is clinically-defined as a ≥20 mmHg decrease in systolic blood pressure and/or a 10 mmHg decrease in diastolic blood pressure when assuming the upright posture. Another critical autonomic issue after SCI is autonomic dysreflexia, which is associated with potentially life threatening elevations in blood pressure, due to afferent input activating sympathetic circuitry located caudally on the spinal cord to the location of the SCI. Clinically, autonomic dysreflexia is defined as elevations in systolic blood pressure of 20 mmHg or more (WO2018148844A1).

WO2018148844A1 discloses a device and algorithm for controlling an autonomic function in an individual. In particular, a controller device is disclosed that utilizes physiological measurements (such as blood pressure) to regulate spinal cord electrical stimulation to stabilize blood pressure. A control interface and algorithm for controlling an autonomic function in a subject. In particular, an algorithm that utilizes physiological measurements is disclosed (such as blood pressure) to regulate spinal cord electrical stimulation to stabilize blood pressure. The neuronal structures involved may be located within the T1 to S5 segments of the spinal cord, for example. Stimulation may be configured to control a particular function by selecting electrodes and/or the nature of the stimulation.

US2007156200A1 discloses an apparatus and a method for controlling blood pressure by stimulating the cardiac afferent sympathetic nerves. The invention may be implemented in a medical device having a pressure sensor for sensing blood pressure, an electrode for providing electrical signals to the cardiac afferent sympathetic nerves, and a controller for providing signals to the electrode as a function of blood pressure signals received from the pressure sensor.

US2011082515A1 discloses a neurostimulation device including an external neurostimulator worn by a patient using a bracing element that braces a portion of the patient's body. The external neurostimulator delivers neurostimulation to modulate a cardiovascular function of the patient. Preferably, the external stimulator delivers the neurostimulation transcutaneously to a stimulation target in the patient's body using surface stimulation electrodes placed on the body approximately over the stimulation target.

US2011/0202107A1 relates to an electric stimulation apparatus for treating hypotension of patients suffering from SCI and a method for treating hypotension. The electric stimulation apparatus comprises: a blood pressure measuring means for continuously measuring a blood pressure of a subject; an electric current application means for intermittently applying an electric current to skin of the subject; and a control means for controlling the electric current application means so as to maintain the blood pressure at a predetermined target blood pressure value by activating the electric current application means when the subject blood pressure is equal to or less than the target blood pressure value.

US2013/0289650A1 relates to neuromodulation for controlling hypertension and other cardio-renal disorders of a patient suffering from SCI. A neuromodulation device is delivered to a patient's body for applying electric activation to decrease renal sympathetic hyperactivity of the patient based on monitored blood pressure of the patient, substantially without thermal energization of the patient's body by applying the electric activation. The electric activation may also depend on monitored blood volume of the patient. A feedback control module may be used to provide feedback control information for adjusting the electric activation based on the monitored blood pressure and volume of the patient.

US3650277A discloses a system for reducing and controlling the blood pressure of a hypertensive patient by providing electrical pulse stimulation of the carotid-sinus nerves controlled by the arterial blood pressure of the patient in such a manner that the number of stimulation pulses within each heart cycle is determined by the arterial means blood pressure whereas the distribution of stimulation pulses over the heart cycle is a function of the arterial pulse wave shape with the pulse frequency being greater during the first portion of the heart cycle.

US6058331 discloses techniques for therapeutically treating peripheral vascular disease. A sensor is implemented for sensing the extent of blood flow in a patient's limb or ischemic pain and generating a corresponding sensor signal. The signal is processed to determine the level of spinal cord stimulation or peripheral nerve stimulation to be applied. This information is provided to a signal generator which thereby provides electrical stimulation energy to one or more stimulation leads. Stimulation of the spinal cord, peripheral nerve or neural tissue ganglia thereby improves blood flow, helps restore tissue health and reduces the extent of ischemic pain in the limbs of a peripheral vascular disease patient or organs of other patients. The present invention thereby allows the stimulation to be adjusted automatically to account for changing conditions of the patient throughout the day.

US2007027495A1 relates to an implantable bladder sensor attachable to an exterior surface of a urinary bladder to sense bladder condition or activity for urinary incontinence, or an inability to control urinary function. The sensor includes a strain gauge that detects mechanical deformation of the bladder. Mechanical deformation may be indicative of a gradual filling of the bladder, or an instantaneous contraction indicating an imminent urine voiding event. Wireless telemetry circuitry within the sensor transmits information to implanted electrical stimulator that delivers electrical stimulation for alleviating urinary incontinence, or to an external programmer that controls the implanted stimulator.

There is a need for a new therapy to better control and/or manage autonomic dysfunction of subjects after SCI.

In the acute phase after injury, optimization of hemodynamics using EES could reduce the number of hypoperfusions experienced by the patients, which previous work has linked directly to the potential for subsequent neurological improvements. However, manual monitoring of hemodynamics 24 hours per day is not realistic in common clinical settings. Therefore, a closed-loop solution to manage blood pressure in both the acute and chronic phases after injury could not only improve patient quality of life, but perhaps even increase the chance of positive neurological outcome.

It is therefore an object of the present invention to provide a solution for a system and method that can better manage autonomic dysfunction after SCI.

This object is solved by the system according to claim 1. Accordingly, a neuromodulation system, especially a neurostimulation system, for treating a patient, especially for enhancing at least one autonomous function such a blood circulation and/or respiration, wherein the system comprises
- at least one signal input module, which is configured to receive at least one or more signals being indicative for blood circulation, especially being indicative for pulse and/or blood pressure,
- at least one control module, wherein the control module is connected to the signal input module,
wherein the control module is configured to adapt the neurostimulation provided by the neuromodulation system on the basis of the signal(s) received by the signal input module.

The invention is based on the basic idea that a stimulation system has to be provided based on a bottom up understanding of the sympathetic circuitry within the spinal cord, in particular how EES can target this circuitry, the optimal sites of EES for blood pressure regulation, and a deep understanding of the specific dynamics of the entire sympathetic nervous system control. In particular, a stimulation paradigm based on functional and anatomical evidence to stimulate and control blood pressure following both acute and chronic SCI was generated. Further, the dynamics of the sympathetic control system including recordings from the main brainstem control center (rostral ventrolateral medulla), sympathetic outflow from the spinal cord (in the form of renal sympathetic nerve recordings), as well as blood pressure dynamics were studied. In particular, using deep learning approaches it has been found that these dynamics are completely dysregulated after SCI. In particular, the system configured and arranged to replace this dysregulation.

Further, the system may comprise at least one stimulation unit and/or at least one real-time monitoring unit, wherein the at least one real-time monitoring unit comprises at least one sensor.

The stimulation unit may provide stimulation. The stimulation may be provided by electrical stimulation. In particular, the stimulation may be provided by a lead comprising one or multiple electrodes. The lead may be implanted. Alternatively, and/or additionally, transcutaneous stimulation by a lead is also generally possible. Autonomic-optimized stimulation may be generally possible.

Stimulation may be delivered epidurally (by epidural electrical stimulation, EES) and/or subdurally.

The stimulation unit may comprise at least one of a neurostimulator, a neuromodulator and a pulse generator, in particular an implantable pulse generator (IPG). The neurostimulator may be connected to the lead.

In general, stimulation may be delivered to the dorsal aspect of the spinal cord of a mammal. The stimulation may target dorsal roots, dorsal afferent fibres within the dorsal roots and/or intraspinal structures that are connected directly or indirectly to sympathetic preganglionic neurons that affect the function being controlled.

In general, stimulation may be applied with stimulation parameters comprising at least frequency, amplitude and pulse width, wherein the frequency may be 10Hz-10kHz, the amplitude may be 0-1A or 0-15V and the pulse width may be 1-500µs.

Additionally and/or alternatively, stimulation may be applied by burst train stimulation. Pulse trains called burst train stimulation may be preferred to increase the specificity and comfort. Burst train stimulation may comprise a series of pulses, e.g. 3 to 5 pulses delivered at 200Hz to 700Hz, repeated at the frequency 10-120Hz.

The real-time monitoring unit may comprise at least one sensor. In particular, the real-time monitoring unit may comprise at least one sensor configured and arranged to measure and/or monitor blood pressure and/or perfusion pressure of a patient, in particular spinal cord perfusion pressure. The sensor may generally measure and/or monitor systolic and/or diastolic and/or mean arterial pressure and/or cerebrospinal fluid pressure (and/or also spinal cord perfusion pressure) of the patient. It may be also possible that the sensor also may report pulse rate. Further, the sensor may be configured and arranged to measure and/or monitor a signal and/or a value and/or a marker that correlates with spinal cord oxygenation. The at least one sensor unit may be invasive or non-invasive. In other words, the at least one sensor may be at least partially implantable and/or implanted. Alternatively, the at least one sensor may be not implanted and/or not implantable.

Further, the sensor may monitor cumulative firing rates from any brainstem control area, especially from the rostral ventrolateral medulla.

In particular, the signals being indicative for blood circulation may be signals indicative for blood pressure and/or cumulative firing rates from at least one brainstem control area, especially but not limited to firing rates from the rostral ventrolateral medulla.

The at least one sensor may be a digital or analog sensor system.

It is generally possible, that at least two sensors form a sensor network. A sensor network may generally comprise both at least one at least partially implanted and/or implantable sensor and at least one non-implantable and/or non-implanted sensor.

An implanted and/or implantable sensor and/or a non-implantable and/or non-implanted sensor may be, but is not limited to, an upper arm blood pressure monitor system or a wrist blood pressure monitor system or a finger blood pressure monitor system. The sensor may measure and/or monitor a blood pressure signal indicative for a blood pressure measurement. In general, the at least one sensor may provide continuous monitoring of blood pressure and/or sporadic monitoring of blood pressure and/or measuring or monitoring blood pressure in preset time intervals.

In particular, the blood pressure sensor may be an invasive arterial line. In particular, the invasive arterial line may monitor blood pressure directly and in real-time.

In particular, the signal input module may comprise an input switch module, wherein the input switch module may be configured to switch between signals indicative for blood pressure and cumulative firing rates from at least one brainstem control area, especially but not limited to firing rates from the rostral ventrolateral medulla.

In particular, the signal input module is configured to receive baseline signals, wherein the baseline signals define at least one target value.

The control module is configured to detect differences between the at least one target value and at least one or more signals indicative for blood circulation, wherein the control module is further configured and arranged to adapt neurostimulation based on the differences between the at least one target value and at least one or more signals indicative for blood circulation and/or a signal and/or value and/or a marker that correlates with spinal cord oxygenation.

In particular, after recording a baseline, i.e. a target pressure, i.e. a target value, a perturbation signal may be provided to a patient. The perturbation signal may be provided in the form of negative pressure, a drug, or in a human patient a tilt-test. However, other types of perturbation signals may be generally possible.

A tilt-test is a medical procedure often used to diagnose dysautonomia or syncope.

Patients with symptoms of dizziness or lightheadedness, with or without a loss of consciousness (fainting), suspected to be associated with a drop in blood pressure or positional tachycardia are good candidates for this test.

The procedure tests for causes of syncope by attempting to cause syncope by having the patient lie flat on a special table or bed and then be monitored with ECG and a blood pressure monitor which measure continuously. The table then creates a change in posture from lying to standing.

In particular, lower body negative pressure may be included.

The control module may detect the change in blood pressure. In particular, the control module may detect the change in blood pressure using a moveable parameter to increase or decrease sensitivity.

Further, the control module may implement a controlled increase in stimulation to the stimulation unit in order to increase blood pressure.

The control module comprises a linear proportional control module, wherein the linear proportional control module is configured to modify at least one of amplitude and frequency of a stimulation paradigm in response to the at least one or more signals indicative for blood circulation with a coefficient β that controls the linear proportion with which the amplitude or frequency changes.

In particular, blood pressure may linearly or almost linearly respond to changes in the amplitude of stimulation.

Further, the control module may comprise a forward module, wherein the forward module is configured and arranged to take into account at least one predictive effect of stimulation to adjust coefficient β with a specified time window.

Further, the control module may be configured to comprise stimulation paradigm control parameters, especially minimal or maximal bounds on the stimulation paradigm.

In general, it may be possible to control blood pressure in a closed loop manner, in response to a lower-body negative pressure stimulus.

In particular, the system may be applied to any mammal suffering from SCI.

Further, a neuromodulation system, especially a neurostimulation system for treating a patient is disclosed, especially for enhancing at least one autonomous function such a blood circulation and/or respiration, wherein the system comprises
- At least one sensing element configured to sense a signal indicative for a physiological parameter of a patient,
- At least one control module, wherein the control module is connected to the sensing element,
- At least one spatial mapping module configured to link spatial electrode stimulation configurations targeting the afferent fibers in the dorsal/posterior roots to at least one physiological effect,
- At least one parameter mapping module configured to prepare stimulation parameters for the control module based on input received from the sensing element and/or the spatial mapping module.

The invention is based on the basic idea that a neurostimulation system is provided which specifically targets and modulates sympathetic pre- and post- ganglionic neurons responsible for autonomous control in a way that the system enables precise and optimized control over autonomous control after SCI. In particular, the neurostimulation system enables optimized neurostimulation by identification of a target value of autonomic function, identification of an electrode configuration with optimal effects on autonomous function and at the same time minimal effects on other functions, e.g. muscle function, optimal and location of a stimulation device and optimization of stimulation parameters.

The sensing element may comprise at least one sensor. In particular, the sensing element may comprise at least one sensor configured and arranged to measure and/or monitor blood pressure and/or perfusion pressure of a patient, in particular spinal cord perfusion pressure, and/or spinal cord oxygenation of a patient.

The sensor may generally measure and/or monitor systolic and/or diastolic and/or mean arterial pressure and/or cerebrospinal fluid pressure (and/or also spinal cord perfusion pressure) and/or spinal cord oxygenation of the patient. It may be also possible that the sensor may also report pulse rate. The at least one sensor element may be invasive or non-invasive. In other words, the at least one sensor may be at least partially implantable and/or implanted. Alternatively, the at least one sensor may be not implanted and/or not implantable.

The at least one sensor may be a digital or analog sensor system.

It is generally possible that at least two sensors may from a sensor network. A sensor network may generally comprise both at least one at least partially implanted and/or implantable sensor and at least one non-implantable and/or non-implanted sensor.

An implanted and/or implantable sensor and/or a non-implantable and/or non-implanted sensor may be, but is not limited to, an upper arm blood pressure monitor system or a wrist blood pressure monitor system or a finger blood pressure monitor system. The sensor may measure and/or monitor a blood pressure signal indicative for a blood pressure measurement. In general, the at least one sensor may provide continuous monitoring of blood pressure and/or sporadic monitoring of blood pressure and/or measuring or monitoring blood pressure in preset time intervals.

In particular, the blood pressure sensor may be an invasive arterial line. In particular, the invasive arterial line may monitor blood pressure directly and in real-time.

In general, the sensing element may monitor a physiological signal in real-time or closed to real-time.

In general, the sensing element may be or may comprise an external beat-by beat blood pressure monitor and/or intrathecal catheter and/or a standard brachial blood pressure cuff and/or any type of upper arm blood pressure monitor system and/or any type of wrist blood pressure monitor system and/or any type of finger blood pressure monitor system and/or a oxygenation sensor.

In particular, the system may comprise at least one control module.

The parameter mapping module may optimize stimulation parameters. In particular the parameter mapping module may optimize stimulation parameters by a reinforcement learning model.

In general, stimulation parameters may comprise at least one of frequency, amplitude and pulse width, wherein the frequency may be 10Hz-10kHz, the amplitude may be 0-1A or 0-15V and the pulse width may be 1-500µs.

Additionally and/or alternatively, stimulation may be applied by burst train stimulation. Pulse trains called burst train stimulation may be preferred to increase the specificity and comfort. Burst train stimulation may comprise a series of several pulses, e.g. 3 to 5 pulses delivered at 200Hz to 700Hz, repeated at the frequency 10-120Hz.

In particular, the system may comprise at least one stimulation element comprising at least one electrode array comprising multiple electrodes.

The electrode array may comprise 8-32 electrodes, in particular 16 electrodes. However, every other number of electrodes comprised in the at least one electrode array may be generally possible.

The electrode array may be designed and/or constructed for being capable to target cardiovascular and/or blood pressure hotspots of the spinal segments.

In particular, stimulation may be provided by electrical stimulation. The electrode array may be implanted. Alternatively, and/or additionally, transcutaneous stimulation by an electrode array may generally be possible. Autonomic-optimized stimulation may be generally possible.

Stimulation may be delivered epidurally (by epidural electrical stimulation, EES) and/or subdurally.

Further, the stimulation element may comprise at least one of a neurostimulator, a neuromodulator and a pulse generator, in particular an implantable pulse generator (IPG). The at least one of a neurostimulator, a neuromodulator and a pulse generator, in particular an IPG may be connected to the electrode array.

In general, stimulation may be delivered to the dorsal aspect of the spinal cord of a mammal. The stimulation may target the posterior/dorsal roots, dorsal afferent fibres and/or intraspinal structures that are connected directly or indirectly to sympathetic preganglionic neurons that affect the function being controlled.

In particular, the system may comprise at least one temporal mapping module configured to link temporal electrode stimulation configurations to at least one physiological effect.

In particular, stimulation may be provided in terms of pulse trains, wherein pulse trains may be provided with different temporal arrangements of stimulation events.

The control module may be configured to identify a target value for autonomic function based on a signal provided by the sensing element.

In particular, the target value may be a baseline value.

The spatial mapping module may isolate key electrodes based on anatomical location and a learning procedure initiated at these electrodes to optimize the configuration of the surrounding electrodes.

The spatial mapping module may be configured to perform a reinforcement learning procedure, wherein the reinforcement learning procedure is part of the process to link spatial electrode stimulation configurations targeting the afferent fibers in the dorsal/posterior roots to at least one physiological effect.

The spatial mapping module may be configured to perform a spatial mapping phase for identifying a suitable electrode configuration in terms of selected electrodes and their spatial arrangement in a first step and a parameter mapping phase for adjusting stimulation parameters for the stimulation provided by the selected electrodes in the first step.

It particular, the suitable electrode configuration may be dependent on the exact location of the electrode array and/or the electrodes of the electrode array.

In particular, key electrodes may be isolated based on anatomical location and a reinforcement learning procedure may be initiated at these electrodes to optimize the configuration of the surrounding electrodes. A brief bout of stimulation may be provided, and pressure monitored, with a 'reward' set to an increase in pressure. The model may thus be 'punished' for configurations that do not reliably modify the physiological effect and rewarded for those that do. Additionally, there may be an option to punish the model if the patient finds a configuration to be uncomfortable, or if other side effects are noted (included but not limited to spasm). In doing so, the system may be able to systematically and rapidly identify the optimal electrode configuration.

In particular, the physiological parameter may be at least one of blood pressure of the patient, spinal cord perfusion pressure of the patient, posture of the patient and/or position of the patient and/or spinal cord oxygenation of the patient.

In particular, blood pressure and spinal cord perfusion pressure, respectively, may be expressed as at least one of systolic and/or diastolic and/or mean arterial pressure and/or cerebrospinal fluid pressure (and/or also spinal cord perfusion pressure).

Further, pulse rate of the patient may be additionally and/or alternatively be monitored. Further, the physiological parameter may be additionally and/or alternatively firing rates from any brainstem control area, especially from the rostral ventrolateral medulla.

In particular, the system may be applied to any mammal suffering from SCI.

In other words, the patient may be any mammal suffering from SCI.

According to an example, the use of a neurostimulation system for treating a patient, especially for enhancing at least one autonomous function such a blood circulation and/or respiration is disclosed.

According to an example a method is disclosed, the method characterized in that the method is performed especially with a system disclosed herein.

It is generally possible that the system and method may also be used in an open-loop fashion. In the particular, the method can be a method for neuromodulation, especially for neurostimulation, for treating a patient, especially for enhancing at least one autonomous function such a blood circulation and/or respiration, comprising the steps of
- receiving at least one or more signals being indicative for blood circulation, especially being indicative for pulse and/or blood pressure and/or oxygenation,
- adapting neurostimulation on the basis of the signal(s) received.

Further details and advantages of the present invention shall now be disclosed in connection with the drawings.

It is shown in
- Fig. 1: a schematical overview of an embodiment of the system for neuromodulation and/or neurostimulation according to the present invention, with which the method according to the present invention can be performed;
- Fig. 2: a schematical overview of a further embodiment of the system 110 for neuromodulation and/or neurostimulation, for the treatment of a patient, according to the present invention, with which the method according to the present invention can be performed;
- Fig. 3: an example of the bottom up understanding of the sympathetic circuitry within the spinal cord;
- Fig. 4a: a general layout of the linear relationship between blood pressure and amplitude of epidural electrical stimulation;
- Fig. 4b: a general layout of the linear relationship between blood pressure and amplitude of epidural electrical stimulation in a non-human primate;
- Fig. 4c: a general layout of the linear relationship between blood pressure and amplitude of epidural electrical stimulation in a human patient;
- Fig. 5a: an example of closed-loop control of blood pressure according to the present invention;
- Fig. 5b: an example of closed-loop control of blood pressure according to the present invention as shown Fig. 5a, here with Acute and Chronic SCI;
- Fig. 5c: an example of closed-loop control of blood pressure according to the present invention in non-human primates;
- Fig. 5d: an example of closed-loop control of blood pressure according to the present invention in a human patient;
- Fig. 6a: a schematical overview of the activation of sympathetic neurons;
- Fig. 6b: a schematical overview of mechanisms, by which EES stabilizes hemodynamics;
- Fig. 7: a schematical overview of an embodiment of the system for neuromodulation and/or neurostimulation according to the present invention, with which the method according to the present invention can be performed;
- Fig. 8a: a graph with a series of 24 spatial electrode configurations Config and the immediate blood pressure response of a patient equipped with the system disclosed in Fig. 7;
- Fig. 8b: a graph of two selected spatial electrode configurations Config disclosed in Fig. 8a and the immediate blood pressure response of a patient equipped with the system 10 disclosed in Fig. 7;
- Fig. 8c: a schematical overview of electrode arrays A with electrode configurations Config according to Fig. 8b;
- Fig. 8d: a further overview of the stimulation effect on blood pressure;
- Fig. 9: shows embodiments, where the stimulus changing blood pressure is constant or variable;
- Fig. 10: shows a blood pressure collapse, which is treated and rescued by the system according to the present invention.

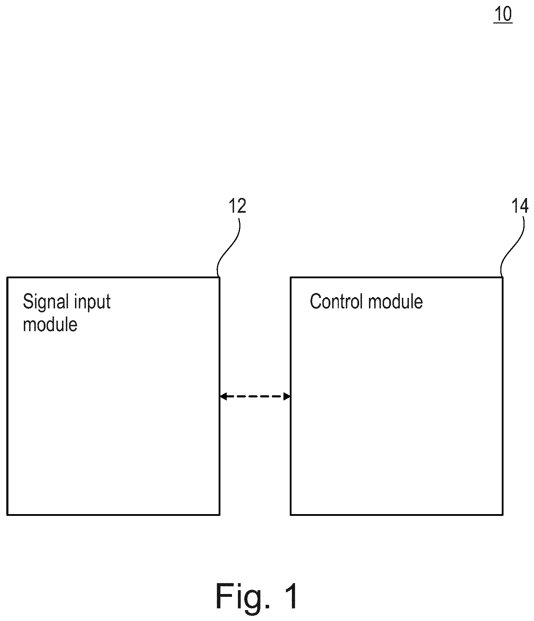

**Fig. 1** shows a schematical overview of an embodiment of the system 10 for neuromodulation and/or neurostimulation, for the treatment of a patient, according to the present invention, with which the method according to the present invention can be performed.

In this embodiment, the system 10 is configured or treating a patient, especially for enhancing at least one autonomous function such as blood circulation and/or respiration.

In this embodiment, the system 10 is configured for treating a patient, especially for enhancing blood pressure function.

Alternatively, and/or additionally, the system 10 could be configured for treating a patient, especially for enhancing any type of autonomous function.

In this embodiment, the system 10 comprises a signal input module 12.

It is generally possible that the signal input module is configured to receive at least one or more signals being indicative for blood circulation, especially being indicative for pulse and/or blood pressure and/or oxygenation.

In this embodiment, the signal input module 12 is configured to receive at least one or more signals being indicative for blood pressure.

It is generally possible that the signal input module 12 is configured to receive additionally and/or alternatively at least one or more signals being indicative for pulse.

In this embodiment, the system 10 further comprises a control module 14.

The control module 14 is connected to the signal input module 12.

In this embodiment, the connection between the control module 14 and the signal input module 12 is a direct and bidirectional connection.

In general, also an indirect and/or unidirectional connection would be generally possible.

In this embodiment, the connection between the control module 14 and the signal input module 12 is a wireless connection.

In general, also cable-bound connection would be generally possible.

In this embodiment, the control module 12 is configured to adapt neurostimulation provided by the neurostimulation system 10 on the basis of the signals received by the signal input module 12.

Not shown in Fig. 1 is that the at least one or more signals being indicative for blood pressure could comprise at least one of diastolic blood pressure, systolic blood pressure, diastolic blood pressure and a systolic blood pressure, mean arterial pressure, blended blood pressure value or the like.

Not shown in Fig. 1 is that the at least one or more signals being indicative for blood circulation could comprise perfusion pressure, i.e. spinal cord perfusion pressure, which is defined as the difference between mean arterial blood pressure and cerebrospinal fluid pressure may be of importance. Further, also the signal can be related to or indicative for oxygenation.

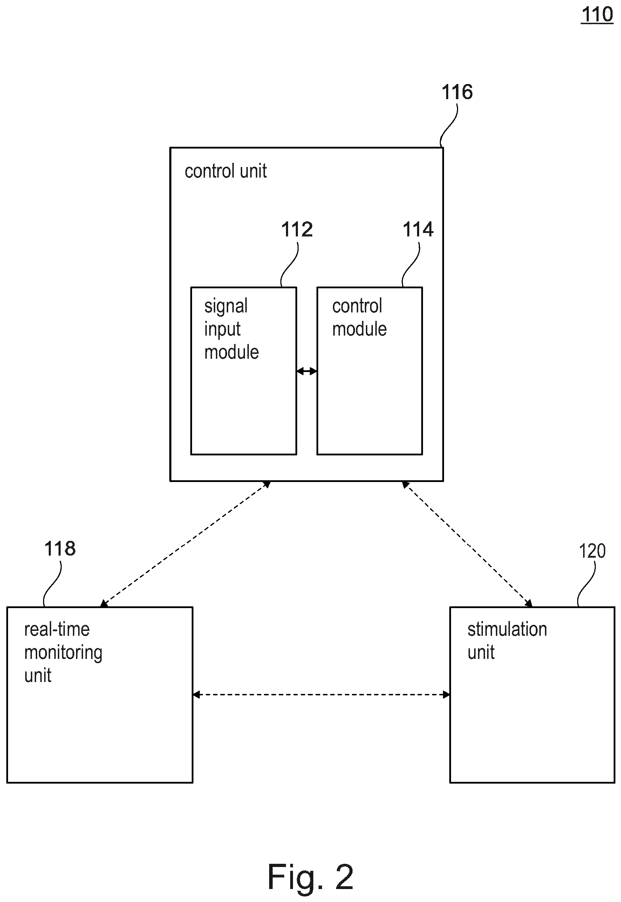

**Fig. 2** shows a schematical overview of a further embodiment of the system 110 for neuromodulation and/or neurostimulation, for the treatment of a patient, according to the present invention, with which the method according to the present invention can be performed.

The system 110 comprises the structural and functional features as disclosed for neuromodulation system 10 in Fig. 1.

The corresponding references are indicated as 100+x (e.g. input module 112).

In this embodiment, the system 110 is configured or treating a patient, especially for enhancing at least one autonomous function of a patient.

In this embodiment, the system 110 is configured for treating a patient, especially for enhancing blood circulation function of a patient.

In this embodiment, the system 110 is configured for treating a patient, especially for enhancing blood pressure function of a patient.

Alternatively, and/or additionally, the system 110 could be configured for treating a patient, especially for enhancing any type of autonomous function of a patient.

The system 110 comprises a control unit 116.

In this embodiment, the control unit 116 comprises a signal input module 112 and a control module 114, cf. signal input module 12 and control module 14 as disclosed in Fig. 1.

The system 110 also comprises a real-time monitoring unit 118.

In this embodiment, the real-time monitoring unit 118 is configured and arranged to monitor blood pressure.

Not shown in this embodiment is that the real-time monitoring unit 118 comprises a sensor.

Not shown in this embodiment is that the sensor is configured and arranged to measure and/or monitor blood pressure of a patient P.

Further, the system 110 comprises a stimulation unit 120.

The stimulation unit 120 is configured and arranged to provide stimulation.

Not shown in Fig. 2 is that stimulation is provided by electrical stimulation.

Not shown in Fig. 2 is that the stimulation unit 120 is constructed to comprise a lead.

In particular, stimulation is provided by a lead comprising one or multiple electrodes.

Not shown in Fig. 2 is that the lead is implanted.

Not shown in Fig. 2 is that alternatively, and/or additionally, transcutaneous stimulation by a lead could generally also be possible.

Not shown in Fig. 2 is that autonomic-optimized stimulation could be generally possible.

Not shown in Fig. 2 is that stimulation could be delivered epidurally (by epidural electrical stimulation, EES) and/or subdurally.

Not shown in Fig. 2 is that the stimulation unit 120 comprises a pulse generator, in particular an implantable pulse generator (IPG).

Not shown in Fig. 2 is that the IPG is connected to the lead.

Not shown in Fig. 2 is that in general, stimulation could be delivered to the dorsal aspect of the spinal cord of a mammal.

Not shown in Fig. 2 is that in general, stimulation could be applied with stimulation parameters comprising at least frequency, amplitude and pulse width, wherein the frequency could be 10Hz-10kHz, the amplitude could be 0-1A or 0-15V and the pulse width could be 1-500µs.

Not shown in Fig. 2 is that in general, stimulation could be applied by burst train stimulation.

Pulse trains called burst train stimulation could be preferred to increase the specificity and comfort.

Burst train stimulation could comprise a series of several pulses, e.g. 3 to 5 pulses delivered at 200Hz to 700Hz, repeated at the frequency 10-120Hz.

In this embodiment, the real-time monitoring unit 118 is configured and arranged to monitor blood pressure.

In this embodiment, the real-time monitoring unit 118 comprises a single sensor.

In an alternative embodiment, the real-time monitoring unit 118 could comprise more than one sensor.

In an alternative embodiment, the real-time monitoring unit 118 could comprise a sensor network.

The sensor is configured and arranged to measure and/or monitor blood pressure of a patient.

In an alternative embodiment, the system 110 may comprise more than one control unit 116 and/or more than one stimulation unit 120 and/or more than one real-time monitoring unit 180.

In this embodiment, the control unit 116 is connected to the stimulation unit 120 and the real-time monitoring unit 118.

In this embodiment, the connection between the control unit 116 and the stimulation unit 120 and the control unit 116 and the real-time monitoring unit 118 is a direct and bidirectional connection.

In this embodiment, the connection between the control unit 116 and the stimulation unit 120, the control unit 116 and the real-time monitoring unit 118 is established by a wireless link.

However, alternatively, also a cable bound and/or unidirectional and/or indirect connection between the control unit 116 and the stimulation unit 120 and the control unit 116 and the real-time monitoring unit 118 could be generally possible.

In this embodiment, the stimulation unit 120 is connected to the real-time monitoring unit 118.

The connection between the stimulation unit 120 and the real-time monitoring unit 118 is a direct and bidirectional connection.

The connection between the stimulation unit 120 and the real-time monitoring unit 118 is established by a wireless link.

However, alternatively, also a cable bound and/or unidirectional and/or indirect connection between the stimulation unit 120 and the real-time monitoring unit 118 could be generally possible.

The real-time monitoring unit 118, in particular the sensor of the real-time monitoring unit 180 measures blood pressure of the patient.

Not shown in this embodiment is that the sensor could generally measure and/or monitor systolic and/or diastolic and/or mean arterial pressure.

Not shown in this embodiment is that a further sensor could additionally and/or alternatively measure cumulative firing rates from a brainstem control area.

Not shown in this embodiment is that a further sensor could additionally and/or alternatively measure cumulative firing rates from the ventrolateral medulla.

In other words, the signals indicative for blood circulation could be signals indicative for blood pressure and/or cumulative firing rates from at least one brainstem control area, especially but. Not limited to firing rates from the rostral ventrolateral medulla.

Not shown in this embodiment is that the sensor could also report pulse rate.

Not shown in this embodiment is that the at least one sensor may be an invasive or non-invasive sensor.

Not shown in this embodiment is that the sensor could be at least partially implantable and/or implanted.

Alternatively, the at least one sensor could be not implantable and/or not implanted.

The measured blood pressure is communicated from the real-time monitoring unit 118 to the signal input module 112.

Not shown in Fig. 2 is that the signal input module 112 could comprise an input switch module, wherein the input switch module could be configured to switch between signals indicative for blood pressure and cumulative firing rates from at least one brainstem control area, especially but not limited to firing rates from the rostral ventrolateral medulla.

Not shown in Fig. 2 is that the signal input module 112 is configured to receive baseline signals, wherein the baseline signals define at least one target value.

Not shown in Fig. 2 is that the control module 114 is configured to detect differences between the at least one target value and at least one or more signals indicative for blood circulation, wherein the control module 114 is further configured and arranged to adapt neurostimulation based on the differences between the at least one target value and at least one or more signals indicative for blood circulation.

Not shown in Fig. 2 is that after recording a baseline, i.e., a target pressure, i.e. a target value, a perturbation signal could be provided to the patient.

The perturbation signal could be provided in the form of negative pressure, a drug, or a tilt-test.

However, any other form of perturbation signal could be generally possible.

In this embodiment, the control module 114 could detect the change in blood pressure.

In particular, the control module 114 could detect the change in blood pressure using a moveable parameter to increase or decrease sensitivity.

In this embodiment, the control module 114 could implement a controlled increase in stimulation to the stimulation unit 120 in order to increase blood pressure.

Not shown in this embodiment is that the control module 114 comprises a linear proportional control module, wherein the linear proportional control module is configured to modify at least one of amplitude and frequency of a stimulation paradigm in response to the at least one or more signals indicative for blood circulation with a coefficient β that controls the linear proportion with which the amplitude or frequency changes.

Further not shown in Fig. 2 is that the control module could comprise a forward module, wherein the forward module is configured and arranged to take into account at least one predictive effect of stimulation to adjust coefficient β with a specified time window.

Not shown in Fig. 2 is that it is generally possible that blood pressure responds linearly or almost linearly to changes in the amplitude of stimulation.

Also not shown in Fig. 2 is that the control module 114 could be configured to comprise stimulation paradigm control parameters, especially minimal or maximal bounds on the stimulation paradigm.

Not shown in Fig. 2 is that it is generally possible that the system 110 is applied to any mammal suffering from SCI.

According to the present invention the use of a system 10, 110 for neuromodulation is disclosed.

The use of the system 10, 110 and functionality of the system 10, f110 can be described as follows:
Use of a neurostimulation system 10, 110 according to the system of any of claims 1 to 9 for treating a patient, especially for enhancing at least one autonomous function such a blood circulation and/or respiration.

In other words, according to the present invention, the use of a neurostimulation system 10, 110 according to the system 10, 110 of any of claims 1 to 9 for treating a patient, especially for enhancing at least one autonomous function such a blood circulation and/or respiration is disclosed.

According to an example a method is disclosed, the method characterized in that the method is performed with a system disclosed herein.

The method performed with the system 10,110 and functionality of the system 10,110 can be described as follows:
A method for neuromodulation, especially for neurostimulation, for treating a patient, especially for enhancing at least one autonomous function such a blood circulation and/or respiration, comprising the steps of
- receiving at least one or more signals being indicative for blood circulation, especially being indicative for pulse and/or blood pressure and/or oxygenation,
- adapting neurostimulation on the basis of the signal(s) received.

In particular, the method could further comprise the steps of providing neurostimulation and monitoring signals indicative for blood circulation in real-time, especially signals being indicative for pulse and/or blood pressure.

In particular, the signals being indicative for blood circulation could be signals indicative for blood pressure and/or cumulative firing rates from at least one brainstem control area, especially but not limited to firing rates from the rostral ventrolateral medulla.

In particular, the method could comprise switching between signals indicative for blood pressure and cumulative firing rates from at least one brainstem control area, especially but not limited to firing rates from the rostral ventrolateral medulla.

In particular, the method could comprise receiving baseline signals, wherein the baseline signals define at least one target value.

Further, the method could be configured for detecting differences between the at least one target value and at least one or more signals indicative for blood circulation, wherein the method could be further configured and arranged to adapt neurostimulation based on the differences between the at least one target value and at least one or more signals indicative for blood circulation and/or oxygenation.

Further, the method could be configured to modify at least one of amplitude and frequency of a stimulation paradigm in response to the at least one or more signals indicative for blood circulation with a coefficient β that controls the linear proportion with which the amplitude or frequency changes.

Further, the method could be configured to take into account a predictive effect of stimulation to adjust coefficient β with a specified time window.

Further, the method could be configured to comprise stimulation paradigm control parameters, especially minimal or maximal bounds on the stimulation paradigm.

Of note, the present system 10 and method could also be applied for the treatment of a mammal suffering from neurological conditions other than SCI, including but not limited to stroke, multiple sclerosis, autonomic failure, autonomic neuropathy, as well as cancer of the neurological tissue which impair operation of descending sympathetic pathways that normally facilitate control of autonomic functions.

Not shown in Fig. 1 is that the present system and method could also be applied for the treatment of any other autonomic dysfunction than impaired blood pressure control, including but not limited to heart rate, digestive function, bladder control and/or bowel control.

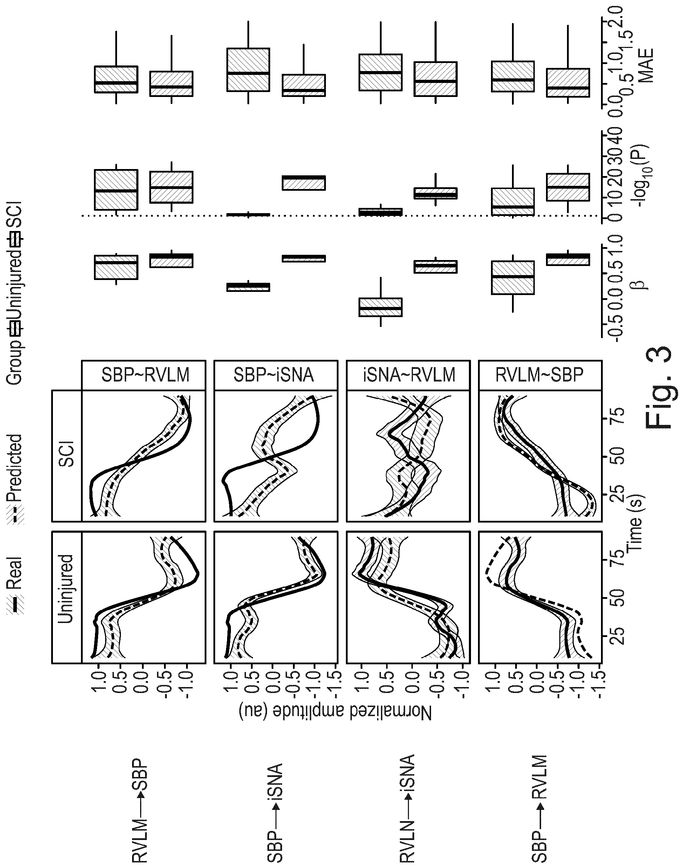

**Fig. 3** shows an example of bottom up understanding of the sympathetic circuitry within the spinal cord.

Deep learning reveals disrupted dynamics between critical control centers, i.e. rostral ventrolateral medulla RVLM, integrated sympathetic nerve activity iSNA, systolic blood pressure SBP, after spinal cord injury SCI.

On the left the specific interaction being studied is described.

The normalized real response (real) to a perturbation that lowers blood pressure, and the predicted response based on machine learning (predicted) was observed.

For uninjured panels a good prediction across each control node in the system exists.

After SCI there is a disruption in the relationship between all aspects and the spinal cord, showing disrupted control.

The system 10, 110 seeks to replace this disrupted control.

The mean absolute error MAE from the deep learning predictions is indicated.

Beta β of a linear regression model is indicated.

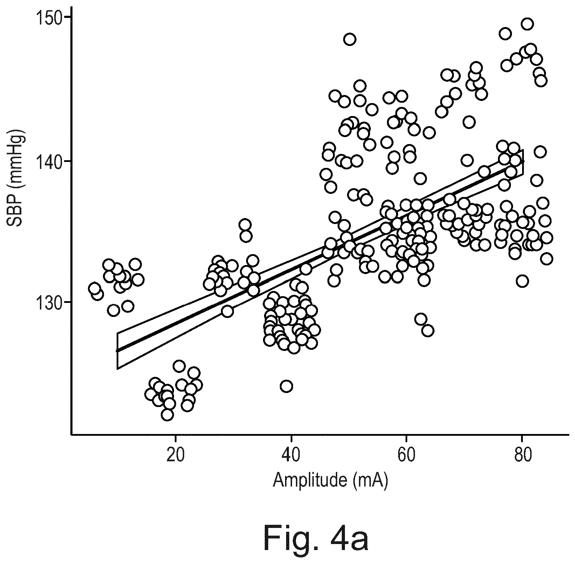

**Fig. 4a** shows a general layout of the linear relationship between blood pressure and amplitude of epidural electrical stimulation.

In particular, the linear relationship between blood pressure and amplitude of epidural electrical stimulation EES provides the basis for a linear proportional control mechanism.

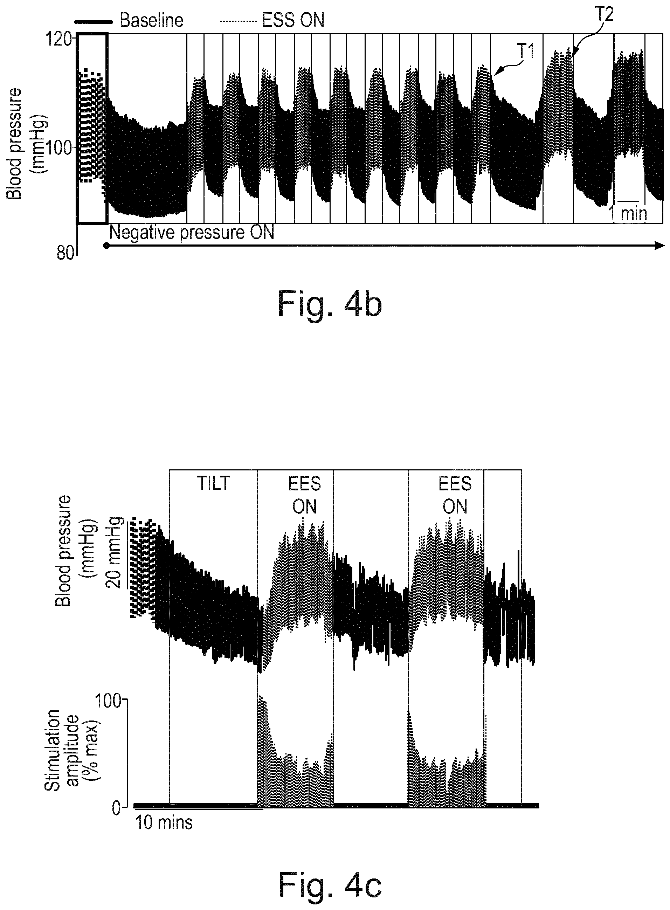

**Fig. 4b** (like Fig. 4a) shows a general layout of the linear relationship between blood pressure and amplitude of epidural electrical stimulation in a non-human primate.

**Fig. 4c** shows a general layout of the linear relationship between blood pressure and amplitude of epidural electrical stimulation in a human patient;

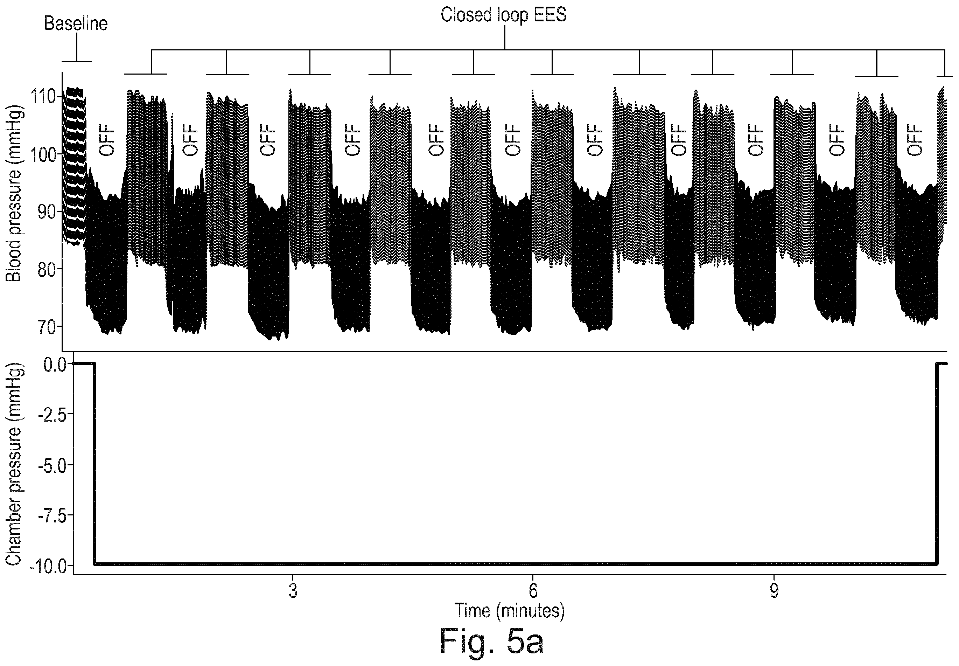

**Fig. 5a** shows an example of closed-loop control of blood pressure according to the present invention.

In this embodiment a patient suffering from SCI is equipped with the system 10, 110 as disclosed in Fig. 1 and/or Fig. 2.

Resting blood pressure was identified (baseline).

Baseline blood pressure was identified as target pressure.

An orthostatic challenge stimulus was identified which continued for 10 minutes (bottom trace for the pressure inside the chamber).

Closed-loop epidural electrical stimulation EES was turned on, consistently reaching the target pressure.

The following parameters were used for stimulation: amplitude control; 50 Hz stimulation; beta = 10; pulse width = 100 micro seconds.

Note that every other parameter could be generally used for stimulation.

In general, the frequency may be 10Hz-10kHz, the amplitude may be 0-1A or 0-15V, and the pulse width may be 1-500µs.

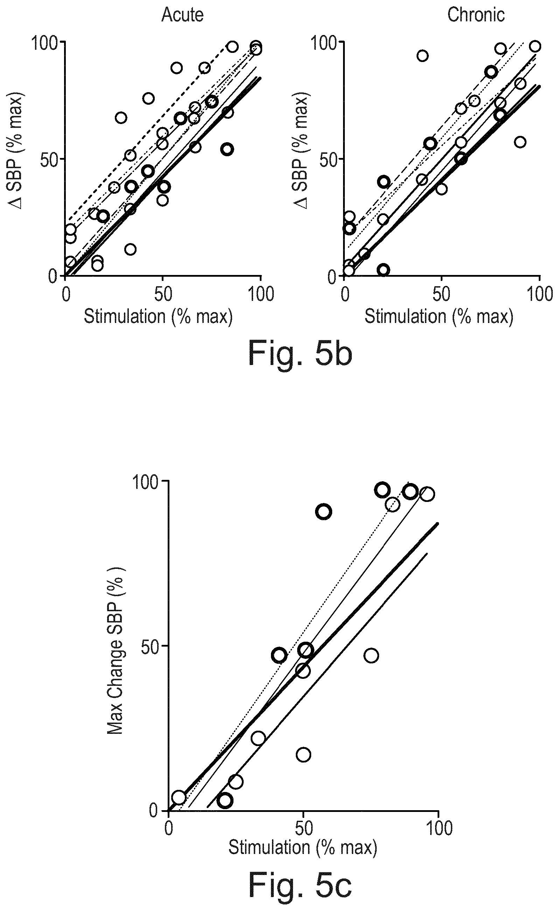

**Fig. 5b** (similar to Fig. 5a) shows an example of closed-loop control of blood pressure according to the present invention as shown Fig. 5a, here with Acute and Chronic SCI;

**Fig. 5c** (similar to Fig. 5a) shows an example of closed-loop control of blood pressure according to the present invention in non-human primates;

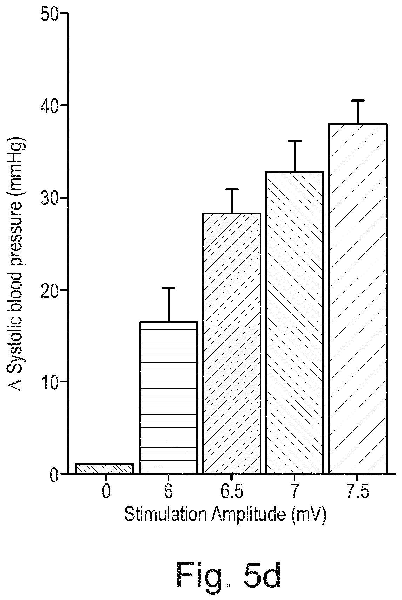

**Fig. 5d** (similar to Fig. 5a) shows an example of closed-loop control of blood pressure according to the present invention in a human patient;

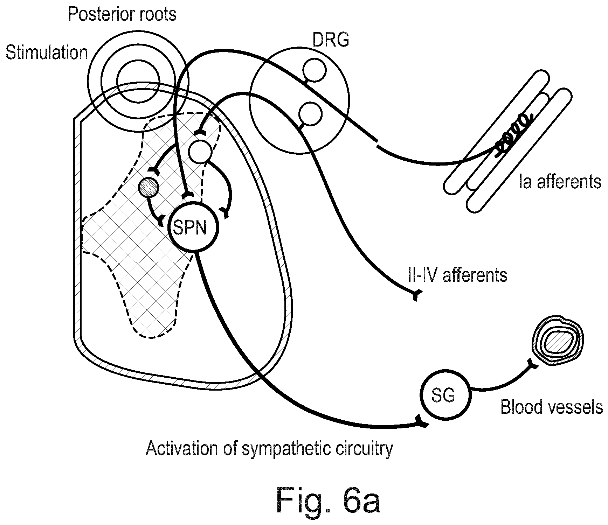

**Fig. 6a** shows a schematical overview of the activation of sympathetic neurons according to the present invention.

In particular, the activation of the sympathetic circuitry in response to stimulation is shown.

In particular, the activation of the sympathetic circuitry in response to stimulation with the system 10, 110 and/or the method according to the present invention is shown.

Stimulation enters through the posterior roots of the dorsal root ganglion DRG and activates sympathetic pre-ganglionic neurons SPN, which then activate splanchic ganglia SG and blood vessels responsible for blood pressure.

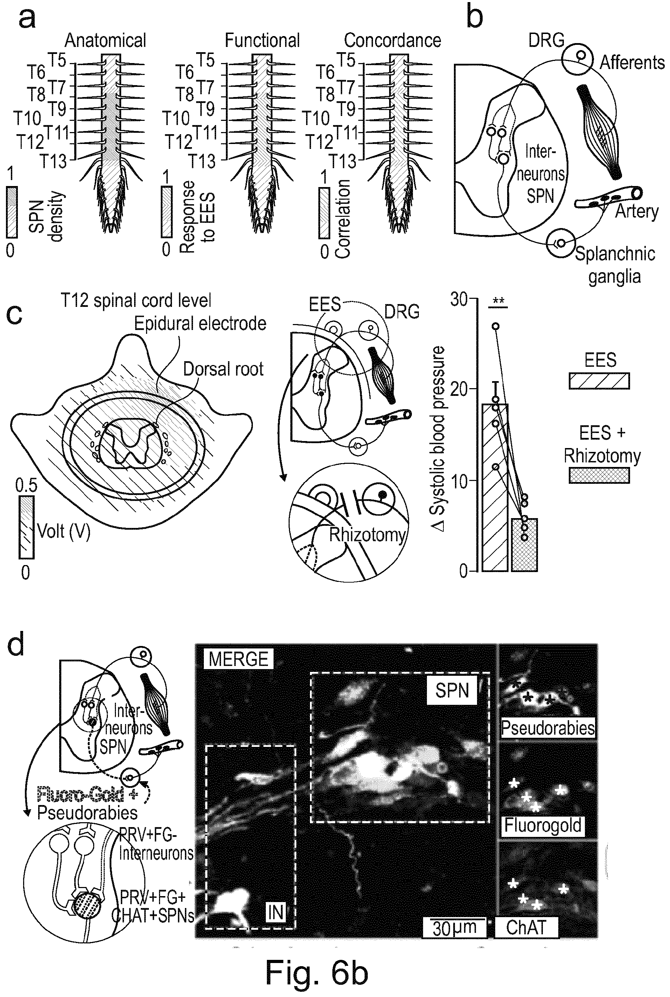

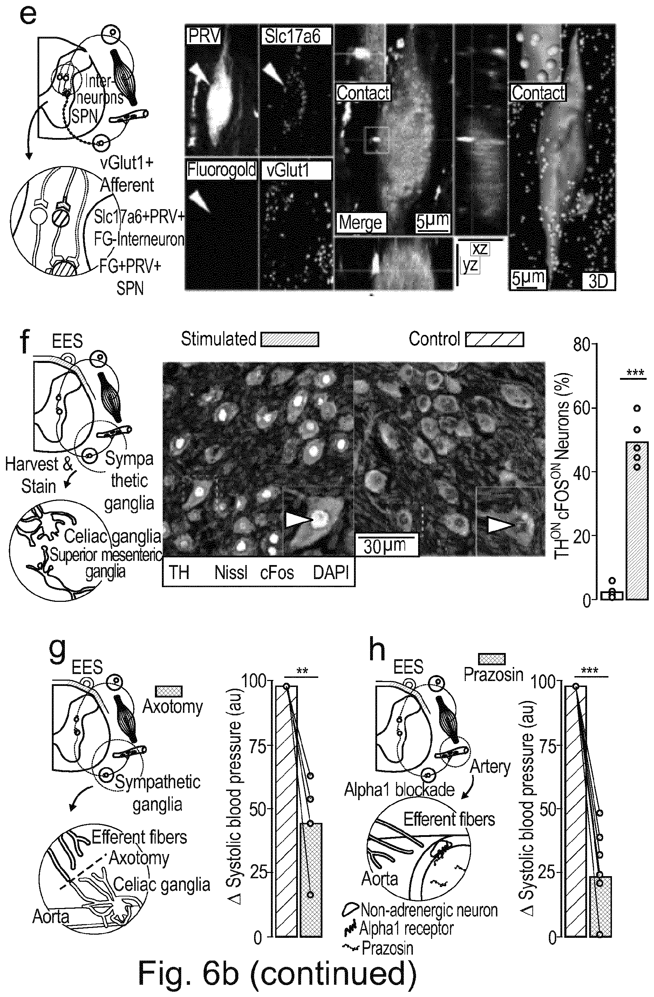

**Fig.** 6b shows a schematical overview of mechanisms, by which EES stabilizes hemodynamics.

**Part a** shows the intraspinal density of neurons retrogradely traced from the splanchnic ganglia, amplitude of pressor responses to TESS applied to each segment, and concordance between anatomical and functional datasets.

**Part b** shows hypothetical circuits activated by TESS to elicit blood vessel constriction. **Part c** shows color-coded electrical potentials following TESS applied to the spinal cord suggesting the exclusive activation of afferent fibres. Scheme illustrating rhizotomy of posterior roots. Barplots report pressor responses to Targeted Epidural Spinal Stimulation (TESS) before and after rhizotomy (n = 5, paired samples one-tailed t-test; t = 4.36; P = 0.006). **Part d** shows trans-synaptic retrograde tracing revealing interneurons connected to splanchnic ganglia.

**Part e** shows interneurons. These interneurons express the excitatory marker Slc17a6, and receive vGlut1 synapses from large-diameter proprioceptive afferents. **Part f** shows Fos expression in THON neurons in the splanchnic ganglia in control and after TESS. Barplot reports percentage of FOSON neurons (n = 5, independent samples one-tailed t-test; t = 13.96; P = 4.99e-05). **Part g** shows ablation of splanchnic efferents blunted the pressor response (n = 4, independent samples one-tailed t-test; t = -4.54; P = 0.0099). **Part h** shows alpha1 receptor blockade with prazosin blunted pressor responses (n = 5, independent samples one-tailed t-test; t = -5.59; P = 0.0007).

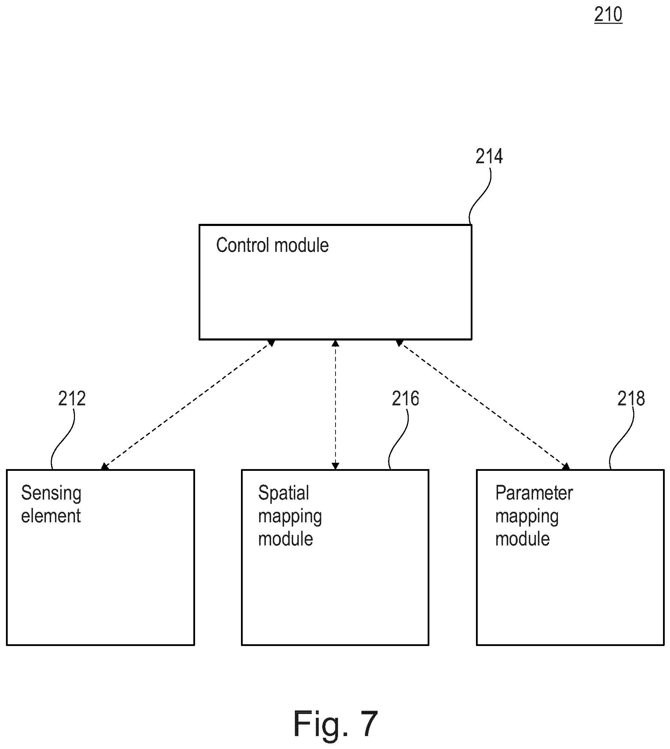

**Fig. 7** shows a schematical overview of a further embodiment of the system 210 for neuromodulation and/or neurostimulation, for the treatment of a patient, according to the present invention, with which the method according to the present invention can be performed.

The system 210 is neurostimulation system 210 for treating a patient, especially for enhancing at least one autonomous function such as blood circulation and/or respiration.

In this embodiment, the system 210 is a neurostimulation system 210 for treating a patient, especially for enhancing blood pressure function.

The system 210 comprises a sensing element 212.

In general, the sensing element 212 is configured to sense a signal indicative for a physiological parameter of a patient.

The system 210 further comprises a control module 214.

In this embodiment, the control module is configured to identify a target value for autonomic function based on a signal provided by the sensing element 212.

The system 210 further comprises a spatial mapping module 216.

In general, the spatial mapping module 216 is configured to link spatial electrode stimulation configurations Config targeting the afferent fibers in the dorsal/posterior roots to at least one physiological effect.

The system 210 further comprises a parameter mapping module 218.

In general, the parameter mapping module 218 is configured to prepare stimulation parameters for the control module 214 based on input received from the sensing element 212 and/or the spatial mapping module 216.

In an alternative embodiment, the system 210 comprises more than one sensing element 212 and/or more than one control module 214 and/or more than one spatial mapping module 216 and/or more than one parameter mapping module 218.

Not shown in Fig. 7 is that the system 210 further comprises at least one stimulation element.

Not shown in Fig. 7 is that the at least one stimulation element comprises at least one electrode array A comprising multiple electrodes E.

Not shown in Fig. 1 is that the electrode array A comprises in this embodiment 216 electrodes E.

In another embodiment, the electrode array A comprises 8-32 electrodes E.

However, any other number of electrodes E is generally possible.

In this embodiment, the control module 214 is connected to the sensing element 212.

The connection between the control module 214 and the sensing element 212 is a direct and bidirectional connection.

However, also an indirect and/or unidirectional connection would be generally possible.

In this embodiment, the connection between the control module 214 and the sensing element 212 is a wireless connection.

However, also a cable-bound connection would be generally possible.

In this embodiment, the control module 214 is connected to the spatial mapping module 216 and the parameter mapping module 218.

The connection between the control module 214 and the spatial mapping module 216 and the parameter mapping module 218 is a direct and bidirectional connection.

However, also an indirect and/or unidirectional connection would be generally possible.

In this embodiment, the connection between control module 214 and the spatial mapping module 216 and the parameter mapping module 218 is a wireless connection.

However, also a cable-bound connection would be generally possible.

It is generally possible that the sensing element 212, the spatial mapping module 216 and/or the parameter mapping module 218 are directly connected.

It is generally possible that the sensing element 212, the spatial mapping module 216 and/or the parameter mapping module 218 are directly connected by a unidirectional connection.

It is generally possible that the sensing element 212, the spatial mapping module 216 and/or the parameter mapping module 218 are directly connected by a bidrectional connection.

It is generally possible that the sensing element 212, the spatial mapping module 216 and/or the parameter mapping module 218 are directly connected by a wireless connection.

It is generally possible that the sensing element 212, the spatial mapping module 216 and/or the parameter mapping module 218 are directly connected by a cable-bound connection.

The sensing element 212 senses a signal indicative for a physiological parameter of a patient.

In this embodiment, the sensing element 212 is a sensor configured to sense a signal indicative for a physiological parameter of a patient.

In this embodiment, the sensing element 212 is a sensor configured to sense a signal indicative for blood pressure of a patient.

In this embodiment, the sensing element 212 is a sensor configured to sense systolic blood pressure SBP.

However, in an alternative embodiment, the sensing element 212 could be alternatively and/or additionally be configured to sense oxygenation and/or diastolic and/or mean arterial pressure and/or cerebrospinal fluid pressure and/or perfusion pressure, in particular spinal cord perfusion pressure.

In other words, the sensing element 212 could generally sense blood pressure and/or perfusion pressure.

However, in an alternative embodiment, the sensing element 212 could be alternatively and/or additionally be configured to sense posture and/or position.

In other words, the physiological parameter could be at least one of blood pressure of the patient, spinal cord perfusion pressure of the patient, posture of the patient and/or position of the patient.

In this embodiment, the sensing element 212 is an invasive arterial line.

In particular, the invasive arterial line senses blood pressure directly and in real-time.

In this embodiment the sensing element 212 senses blood pressure continuously.

However, it could be generally possible that the sensing element 212 provides sporadic monitoring of blood pressure and/or monitoring blood pressure in preset time intervals.

Not shown in Fig. 7 is that it could be generally possible that the sensing element 212 could be an implanted and/or implantable sensor and/or a non-implantable and/or non-implanted sensor.

Not shown in Fig. 7 is that could be generally possible that the sensing element 212 could be an external beat-by beat blood pressure monitor, intrathecal catheter and/or a standard brachial blood pressure cuff and/or any type of upper arm blood pressure monitor system and/or any type of wrist blood pressure monitor system and/or any type of finger blood pressure monitor system.

Not shown in Fig. 7 is that the system 210 could comprise at least one temporal mapping module configured to link temporal electrode stimulation configurations to at least one physiological effect.

In this embodiment, the control module 214 identifies a target value for autonomic function based on a signal provided by the sensing element 212.

In this embodiment, the spatial mapping module links spatial electrode stimulation configurations Config to blood pressure, in particular systolic blood pressure SBP.

It is generally possible that the spatial mapping module links spatial electrode stimulation configurations Config to at least one physiological effect.

In this embodiment, the parameter mapping module 218 prepares stimulation parameters for the control module 214 based on input received from the sensing element 212 and/or the spatial mapping module 216.

Not shown in Fig. 7 is that the spatial mapping module 216 could isolate key electrodes E based on anatomical location and a learning procedure initiated at these electrodes E to optimize the configuration of the surrounding electrodes E.

Not shown in Fig. 7 is that the spatial mapping module 216 could be configured to perform a reinforcement learning procedure, wherein the reinforcement learning procedure is part of the process to link spatial electrode stimulation configurations Config targeting the afferent fibers in the dorsal/posterior roots to at least one physiological effect.

Not shown in Fig. 7 is that the spatial mapping module 216 could be configured to perform a spatial mapping phase for identifying a suitable electrode configuration Config in terms of selected electrodes E and their spatial arrangement in a first step and a parameter mapping phase for adjusting stimulation parameters for the stimulation provided by the selected electrodes E in the first step.

Not shown in Fig. 7 is that the stimulation parameters could comprise at least frequency, amplitude and pulse width, wherein the frequency is 10Hz-10kHz, the amplitude is 0-1A or 0-15V and the pulse width is 1-500µs.

Not shown in Fig. 7 is that in general, stimulation could be applied by burst train stimulation. Pulse trains called burst train stimulation could be preferred to increase the specificity and comfort.

Burst train stimulation could comprise a series of several pulses, e.g. 3 to 5 pulses delivered at 200Hz to 700Hz, repeated at the frequency 10-120Hz.

According to an example the use of a system 210 for neuromodulation is disclosed.

The use of the system 210 and functionality of the system 210 can be described as follows:

Use of a neuromodulation system 210 according to the neuromodulation system 210 for treating a patient, especially for enhancing at least one autonomous function such as blood circulation and/or respiration.

Thus, according to an example, the use of a neurostimulation system 210 according to the system 210 for treating a patient, especially for enhancing at least one autonomous function such a blood circulation and/or respiration is disclosed.

According to an example a method is disclosed, the method characterized in that the method is performed with the system 10, 110 and 210 and as defined in the claims.

The method performed with the system 210 and functionality of the system 210 can be described as follows:
The method is a neuromodulation method, especially a neurostimulation method for treating a patient, especially for enhancing at least one autonomous function such a blood circulation and/or respiration, wherein the method comprises at least the following steps:
- Performing a sensing procedure for sensing a signal indicative for a physiological parameter of a patient,
- Performing a spatial mapping procedure to link spatial electrode stimulation configurations Config targeting the afferent fibers in the dorsal/posterior roots to at least one physiological effect,
- Performing a parameter mapping procedure to prepare stimulation parameters based on input received from the sensing and/or the spatial mapping procedure.

In particular, the method could comprise the step of providing stimulation.

Further, the method could comprise the step of linking temporal electrode stimulation configurations to at least one physiological effect.

Further, the method could comprise the step of identifying a target value for autonomic function based on a signal indicative for a physiological parameter of a patient.

Further, the method may comprise the step of isolating key electrodes E based on anatomical location and a learning procedure initiated at these electrodes E to optimize the configuration Config of the surrounding electrodes E.

In general, stimulation parameters could comprise at least frequency, amplitude and pulse width, wherein the frequency could be 10Hz-10kHz, the amplitude could be 0-1A or 0-15V and the pulse width could be 1-500µs.

Further, the method could comprise the step of performing a reinforcement learning procedure, wherein the reinforcement learning procedure is part of the process to link spatial electrode stimulation configurations Config targeting the afferent fibers in the dorsal/posterior roots to at least one physiological effect.

It is generally possible that the method further comprises the step of performing a spatial mapping phase for identifying a suitable electrode configuration Config in terms of selected electrodes E and their spatial arrangement in a first step and a parameter mapping phase for adjusting stimulation parameters for the stimulation provided by the selected electrodes E in the first step.

It is generally possible that the physiological parameter could be at least one of oxygenation (including but not limited to spinal cord oxygenation), blood pressure of the patient, spinal cord perfusion pressure of the patient, posture of the patient and/or position of the patient.

Of note, the present system 10 and method could also be applied for the treatment of a mammal suffering from neurological conditions other than SCI, including but not limited to stroke, multiple sclerosis, autonomic failure, autonomic neuropathy, as well as cancer of the neurological tissue which impair operation of descending sympathetic pathways that normally facilitate control of autonomic functions.

Not shown in Fig. 7 is that the present system and method could be applied for the treatment of any type of autonomic dysfunction including but not limited to heart rate, digestive function, bladder control and/or bowel control.

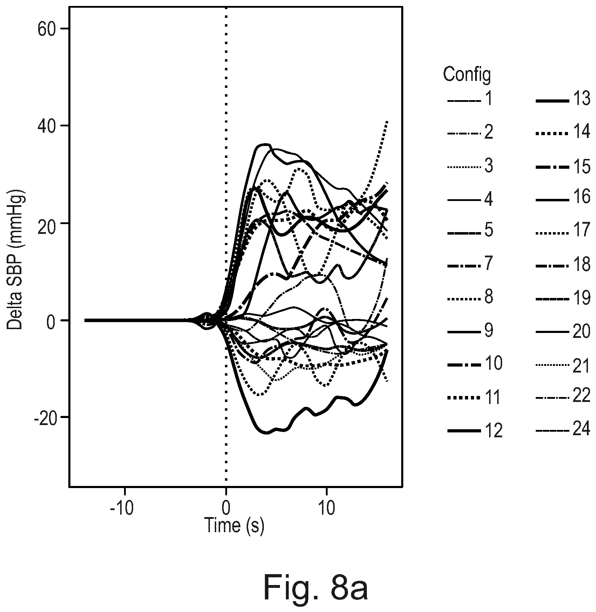

**Fig. 8a****-d** shows exemplary data from a patient equipped with the system 210 according to Fig. 7.

In particular, a patient suffering from spinal cord injury SCI was equipped with the system 210 disclosed in Fig. 7.

In particular, a series of spatial electrode configurations Config is shown, and the immediate blood pressure response, i.e. systolic blood pressure SBP, of the patient was measured, cf. Fig. 8a.

In this embodiment, a series of 24 spatial configurations was assessed.

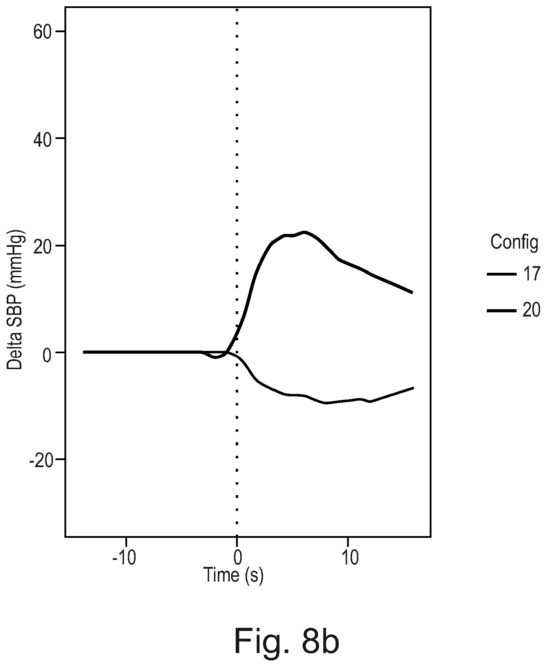

In particular, the optimal spatial electrode configuration Config was selected, based on both the immediate increase in blood pressure and the absence of significant muscle contractions, cf. Fig. 8b.

In this embodiment, the optimal spatial electrode configuration Config selected was configuration Config 20.

In this embodiment, a suboptimal spatial electrode configuration Config would be e.g. spatial configuration Config 17.

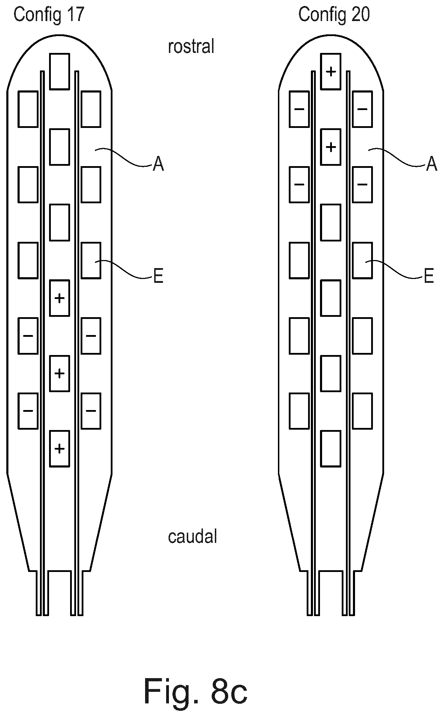

In this example, the rostral four electrodes were identified most optimal to control blood pressure in the patient, cf. Fig. 8c.

The system enabled the identification of this optimized parameters.

Not shown is here that it is generally possible to visualize the electrode array comprising multiple electrodes using a CT or MRI or X-Ray scan to confirm the location of the electrode array.

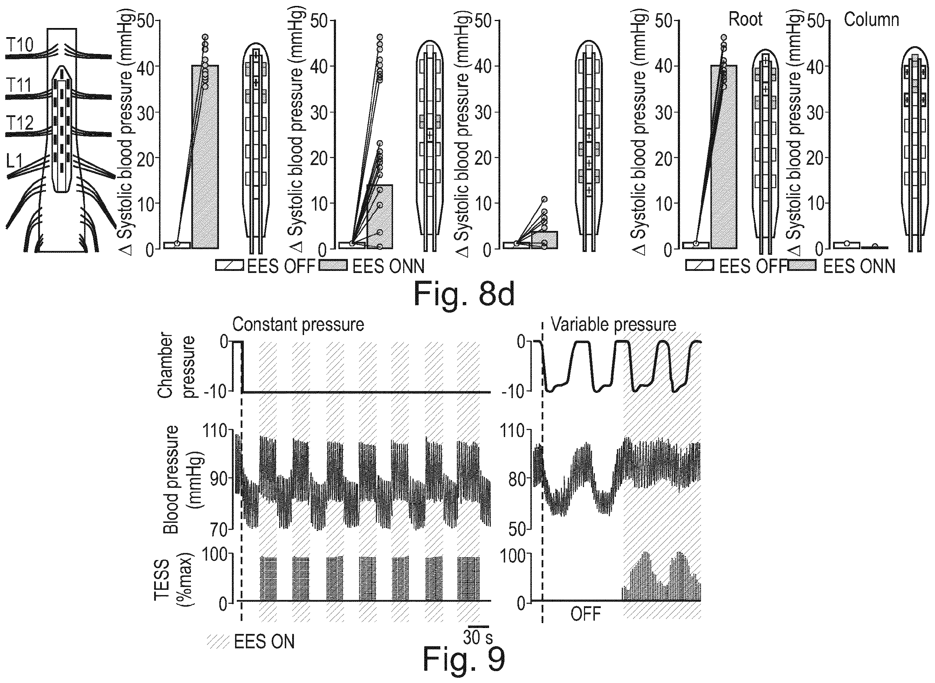

**Fig. 8d** shows a further overview of the stimulation effect on blood pressure.

**Fig. 9** shows embodiments, where the stimulus changing blood pressure is constant or variable.

On the left side, there is pressure in the chamber, blood pressure and TESS amplitude while the neuroprosthetic baroreflex is turned on and off sequentially. On the right side, there are the same variables as in left shown for cyclical changes in the pressure of the chamber.

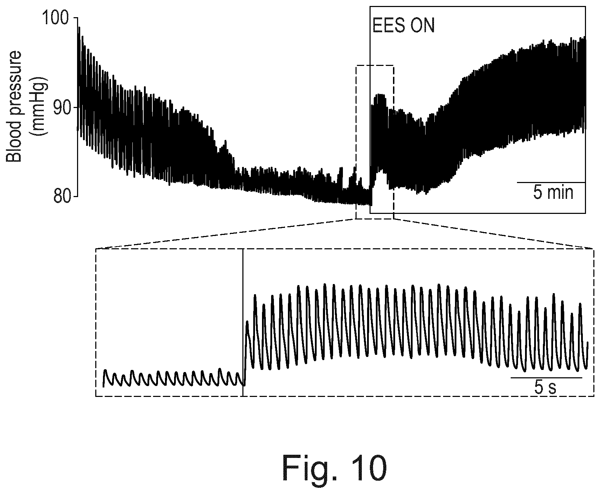

**Fig. 10** shows a blood pressure collapse, which is treated and rescued by the system according to the present invention.

### References

- 10, 110: system
- 12, 112: signal input module
- 14, 114: control module
- 116: control unit
- 118: real-time monitoring unit
- 120: stimulation unit

- 210: system
- 212: control module
- 214: sensing element
- 216: spatial mapping module
- 218: parameter mapping module

- β: coefficient

- A: electrode array
- E: electrode
- Config: spatial electrode configuration
- DRG: dorsal root ganglion
- EES: epidural electrical stimulation
- iSNA: integrated sympathetic nerve activity
- MAE: mean absolute error
- RVLM: rostral ventrolateral medulla
- SBP: systolic blood pressure
- SCI: spinal cord injury
- SPN: sympathetic pre-ganglionic neurons
- SG: splanchic ganglia

## Claims

1. A neuromodulation system (10, 110) for providing epidural stimulation for treating a patient and for enhancing at least one autonomous function such as blood circulation and/or respiration, wherein the system comprises
at least one signal input module (12, 112), which is configured to receive at least one or more signals being indicative for blood circulation,
wherein the at least one signal input module (12, 112) is further configured to receive baseline signals, wherein the baseline signals define at least one target value, and
at least one control module (14, 114), wherein the control module (14, 114) is connected to the signal input module (12, 112),
wherein the control module (14, 114) is configured to adapt the epidural electrical stimulation provided by the neuromodulation system (10, 110) on the basis of the signal(s) received by the signal input module (12, 112), wherein the control module (14, 114) is configured to detect differences between the at least one target value and at least one or more signals indicative for blood circulation, and wherein the control module (14, 114) is further configured and arranged to adapt stimulation based on the differences between the at least one target value and at least one or more signals indicative for blood circulation,
wherein the control module (14, 114) comprises a linear proportional control module configured to provide a linear proportional control mechanism, and wherein the linear proportional control module is further configured to modify at least one of amplitude and frequency of a stimulation paradigm in response to the at least one or more signals indicative for blood circulation with a coefficient β that controls the linear proportion with which the amplitude or frequency changes.

2. The neuromodulation system (10, 110) according to claim 1, **characterized in that** the neuromodulation system (10, 110) further comprises at least one stimulation unit (120) and/or at least one real-time monitoring unit (118), wherein the at least one real-time monitoring unit (118) comprises at least one sensor.

3. The neuromodulation system (10, 110) according to claim 1 or claim 2, **characterized in that** the signals being indicative for blood circulation are signals indicative for oxygenation and/or blood pressure and/or cumulative firing rates from at least one brainstem control area.

4. The neuromodulation system (10, 110) according to claim 1, **characterized in that** the at least one signal input module (12, 112) comprises an input switch module, wherein the input switch module is configured to switch between signals indicative for blood pressure and cumulative firing rates from at least one brainstem control area.

5. The neuromodulation system (10, 110) according to one of the preceding claims, **characterized in that** the control module (14,114) comprises a forward module, wherein the forward module is configured and arranged to take into account at least one predictive effect of stimulation to adjust coefficient β with a specified time window.

6. The neuromodulation system (10, 110) according to one of the preceding claims, **characterized in that** the control module (14, 114) is configured to comprise stimulation paradigm control parameters.

7. The neuromodulation system (210) according to one of the preceding claims, **characterized in that**
the signal input module is or comprises at least one sensing element (212) configured to sense a signal indicative for a physiological parameter of a patient, at least one spatial mapping module (216) configured to link spatial electrode stimulation configurations (Config) targeting the afferent fibers in the dorsal/posterior roots to at least one physiological effect,
at least one parameter mapping module (218) configured to prepare stimulation parameters for the control module (214) based on input received from the sensing element (212) and/or the spatial mapping module (216).

8. The neuromodulation system (10) according to claim 7, **characterized in that** the physiological parameter is at least one of oxygenation, blood pressure of the patient, spinal cord perfusion pressure of the patient, posture of the patient and/or position of the patient.

9. The system (10) according to claim 7, **characterized in that** the system comprises at least one stimulation element comprising at least one electrode array A comprising multiple electrodes E.

10. The system (10) according to claim 9, **characterized in that** the system comprises at least one temporal mapping module configured to link temporal electrode stimulation configurations to at least one physiological effect.

11. The system (10) according to one of claims 9-10, **characterized in that** the control module (14) is configured to identify a target value for autonomic function based on a signal provided by the sensing element (12).

12. The neuromodulation system (10) according to one of claims 9-11, **characterized in that** the spatial mapping module (16) isolates key electrodes E based on anatomical location of the afferent fibers in the dorsal roots and a learning procedure initiated at these electrodes E to optimize the configuration of the surrounding electrodes E.

13. The neuromodulation system (10) according to one of the preceding claims, **characterized in that** the stimulation parameters comprise at least frequency, amplitude and pulse width, wherein the frequency is 10Hz-10kHz, the amplitude is 0-1 A or 0-15V and the pulse width is 1-500µs.

14. The neuromodulation system (10) according to one of the preceding claims, **characterized in that** the spatial mapping module (16) is configured to perform a reinforcement learning procedure, wherein the reinforcement learning procedure is part of the process to link spatial electrode stimulation configurations (Config) targeting the afferent fibers in the dorsal/posterior roots to at least one physiological effect.

15. The neuromodulation system (10) according to one of the preceding claims, **characterized in that** the spatial mapping module (16) is configured to perform a spatial mapping phase for identifying a suitable electrode configuration (Config) in terms of selected electrodes E and their spatial arrangement in a first step and a parameter mapping phase for adjusting stimulation parameters for the stimulation provided by the selected electrodes E in the first step.

## Patentansprüche

1. Neuromodulationssystem (10, 110) zum Bereitstellen einer epiduralen Stimulation zur Behandlung eines Patienten und zur Verbesserung mindestens einer autonomen Funktion wie Blutzirkulation und/oder Atmung, wobei das System umfasst
mindestens ein Signaleingangsmodul (12, 112), das dazu konfiguriert ist, mindestens ein oder mehrere Signale zu empfangen, die auf die Blutzirkulation hinweisen,
wobei das mindestens eine Signaleingangsmodul (12, 112) ferner dazu konfiguriert ist, Basisliniensignale zu empfangen, wobei die Basisliniensignale mindestens einen Zielwert definieren, und
mindestens ein Steuermodul (14, 114), wobei das Steuermodul (14, 114) mit dem Signaleingangsmodul (12, 112) verbunden ist,
wobei das Steuermodul (14, 114) dazu konfiguriert ist, die vom Neuromodulationssystem (10, 110) bereitgestellte epidurale elektrische Stimulation auf der Grundlage der vom Signaleingangsmodul (12, 112) empfangenen Signale anzupassen, wobei das Steuermodul (14, 114) dazu konfiguriert ist, Unterschiede zwischen dem mindestens einen Zielwert und mindestens einem oder mehreren auf die Blutzirkulation hinweisenden Signalen zu erkennen, und wobei das Steuermodul (14, 114) ferner dazu konfiguriert und angeordnet ist, die Stimulation auf der Grundlage der Unterschiede zwischen dem mindestens einen Zielwert und mindestens einem oder mehreren auf die Blutzirkulation hinweisenden Signalen anzupassen,
wobei das Steuermodul (14, 114) ein lineares Proportionalsteuermodul umfasst, das dazu konfiguriert ist, einen linearen Proportionalsteuermechanismus bereitzustellen, und wobei das lineare Proportionalsteuermodul ferner dazu konfiguriert ist, zumindest eines von Amplitude und Frequenz eines Stimulationsparadigmas als Reaktion auf das zumindest eine oder die mehreren Signale, die auf die Blutzirkulation hinweisen, mit einem Koeffizienten β zu modifizieren, der die lineare Proportion steuert, mit der sich die Amplitude oder Frequenz ändert.

2. Neuromodulationssystem (10, 110) nach Anspruch 1,
**dadurch gekennzeichnet, dass** das Neuromodulationssystem (10, 110) ferner mindestens eine Stimulationseinheit (120) und/oder mindestens eine Echtzeitüberwachungseinheit (118) umfasst, wobei die mindestens eine Echtzeitüberwachungseinheit (118) mindestens einen Sensor umfasst.

3. Neuromodulationssystem (10, 110) nach Anspruch 1 oder 2,
**dadurch gekennzeichnet, dass** die Signale, die auf die Blutzirkulation hinweisen, Signale sind, die auf die Sauerstoffsättigung und/oder den Blutdruck und/oder die kumulativen Feuerungsraten aus mindestens einem Hirnstammkontrollbereich hinweisen.

4. Neuromodulationssystem (10, 110) nach Anspruch 1,
**dadurch gekennzeichnet, dass** das mindestens eine Signaleingangsmodul (12, 112) ein Eingangsschaltmodul umfasst, wobei das Eingangsschaltmodul dazu konfiguriert ist, zwischen Signalen umzuschalten, die den Blutdruck und die kumulativen Feuerungsraten von mindestens einem Hirnstammkontrollbereich anzeigen.

5. Neuromodulationssystem (10, 110) nach einem der vorstehenden Ansprüche, **dadurch gekennzeichnet, dass** das Steuermodul (14, 114) ein Vorwärtsmodul umfasst, wobei das Vorwärtsmodul so konfiguriert und angeordnet ist, dass es mindestens einen prädiktiven Effekt der Stimulation berücksichtigt, um den Koeffizienten β innerhalb eines angegebenen Zeitfensters anzupassen.

6. Neuromodulationssystem (10, 110) nach einem der vorstehenden Ansprüche, **dadurch gekennzeichnet, dass** das Steuermodul (14, 114) so konfiguriert ist, dass es Steuerparameter für das Stimulationsparadigma umfasst.

7. Neuromodulationssystem (210) nach einem der vorstehenden Ansprüche, **dadurch gekennzeichnet, dass**
das Signaleingabemodul mindestens ein Sensorelement (212) ist oder umfasst, das so konfiguriert ist, dass es ein Signal erfasst, das einen physiologischen Parameter eines Patienten anzeigt, mindestens ein räumliches Abbildungsmodul (216), das so konfiguriert ist, dass es räumliche Elektrodenstimulationskonfigurationen (Config), die auf die afferenten Fasern in den dorsalen/hinteren Wurzeln abzielen, mit mindestens einem physiologischen Effekt verknüpft,
mindestens ein Parameterabbildungsmodul (218), das dazu konfiguriert ist, Stimulationsparameter für das Steuermodul (214) basierend auf den vom Sensorelement (212) und/oder dem räumlichen Abbildungsmodul (216) empfangenen Eingaben vorzubereiten.

8. Neuromodulationssystem (10) nach Anspruch 7,
**dadurch gekennzeichnet, dass** der physiologische Parameter mindestens einer der folgenden ist: Sauerstoffsättigung, Blutdruck des Patienten, Rückenmarksperfusionsdruck des Patienten, Körperhaltung des Patienten und/oder Position des Patienten.

9. System (10) nach Anspruch 7, **dadurch gekennzeichnet, dass** das System mindestens ein Stimulationselement umfasst, das mindestens ein Elektrodenarray A, umfassend mehrere Elektroden E, umfasst.

10. System (10) nach Anspruch 9, **dadurch gekennzeichnet, dass** das System mindestens ein zeitliches Abbildungsmodul umfasst, das so konfiguriert ist, dass es zeitliche Elektrodenstimulationskonfigurationen mit mindestens einem physiologischen Effekt verknüpft.

11. System (10) nach einem der Ansprüche 9 bis 10,
**dadurch gekennzeichnet, dass** das Steuermodul (14) dazu konfiguriert ist, einen Zielwert für die autonome Funktion basierend auf einem vom Sensorelement (12) bereitgestellten Signal zu identifizieren.

12. Neuromodulationssystem (10) nach einem der Ansprüche 9 bis 11, **dadurch gekennzeichnet, dass** das räumliche Abbildungsmodul (16) Schlüsselelektroden E auf der Grundlage der anatomischen Lage der afferenten Fasern in den dorsalen Wurzeln isoliert und an diesen Elektroden E einen Lernvorgang einleitet, um die Konfiguration der umgebenden Elektroden E zu optimieren.

13. Neuromodulationssystem (10) nach einem der vorstehenden Ansprüche, **dadurch gekennzeichnet, dass** die Stimulationsparameter mindestens Frequenz, Amplitude und Impulsbreite umfassen, wobei die Frequenz 10 Hz bis 10 kHz, die Amplitude 0 bis 1 A oder 0 bis 15 V und die Impulsbreite 1 bis 500 µs beträgt.

14. Neuromodulationssystem (10) nach einem der vorstehenden Ansprüche, **dadurch gekennzeichnet, dass** das räumliche Abbildungsmodul (16) so konfiguriert ist, dass es ein Verstärkungslernverfahren durchführt, wobei das Verstärkungslernverfahren Teil des Prozesses ist, um räumliche Elektrodenstimulationskonfigurationen (Config), die auf die afferenten Fasern in den dorsalen/hinteren Wurzeln abzielen, mit mindestens einem physiologischen Effekt zu verknüpfen.

15. Neuromodulationssystem (10) nach einem der vorstehenden Ansprüche, **dadurch gekennzeichnet, dass** das räumliche Abbildungsmodul (16) dazu konfiguriert ist, in einem ersten Schritt eine räumlichen Abbildungsphase zum Identifizieren einer geeigneten Elektrodenkonfiguration (Config) hinsichtlich ausgewählter Elektroden E und ihrer räumlichen Anordnung und in dem ersten Schritt eine Parameterabbildungsphase zum Anpassen der Stimulationsparameter für die von den ausgewählten Elektroden E bereitgestellte Stimulation durchzuführen.

## Revendications

1. Système de neuromodulation (10, 110) destiné à fournir une stimulation épidurale pour traiter un patient et pour améliorer au moins une fonction autonome telle qu'une circulation sanguine et/ou une respiration, dans lequel le système comprend
au moins un module d'entrée de signal (12, 112), configuré pour recevoir au moins un ou plusieurs signaux indiquant la circulation sanguine,
dans lequel au moins un module d'entrée de signal (12, 112) est en outre configuré pour recevoir des signaux de base, dans lequel les signaux de base définissent au moins une valeur cible, et
au moins un module de commande (14, 114), dans lequel le module de commande (14, 114) est connecté au module d'entrée de signal (12, 112),
dans lequel le module de commande (14, 114) est configuré pour adapter la stimulation électrique épidurale fournie par le système de neuromodulation (10, 110) sur la base du ou des signaux reçus par le module d'entrée de signal (12, 112), dans lequel le module de commande (14, 114) est configuré pour détecter des différences entre l'au moins une valeur cible et au moins un ou plusieurs signaux indiquant la circulation sanguine, et dans lequel le module de commande (14, 114) est en outre configuré et agencé pour adapter la stimulation sur la base de différences entre l'au moins une valeur cible et au moins un ou plusieurs signaux indiquant la circulation sanguine,
dans lequel le module de commande (14, 114) comprend un module de commande proportionnelle linéaire configuré pour fournir un mécanisme de commande proportionnelle linéaire, et dans lequel le module de commande proportionnelle linéaire est en outre configuré pour modifier au moins une amplitude et une fréquence d'un paradigme de stimulation en réponse à l'au moins un ou plusieurs signaux indiquant la circulation sanguine avec un coefficient β qui commande la proportion linéaire avec laquelle l'amplitude ou la fréquence change.

2. Système de neuromodulation (10, 110) selon la revendication 1, **caractérisé en ce que** le système de neuromodulation (10, 110) comprend en outre au moins une unité de stimulation (120) et/ou au moins une unité de surveillance en temps réel (118), dans lequel l'au moins une unité de surveillance en temps réel (118) comprend au moins un capteur.

3. Système de neuromodulation (10, 110) selon la revendication 1 ou la revendication 2, **caractérisé en ce que** les signaux indiquant la circulation sanguine sont des signaux indiquant une oxygénation et/ou une pression artérielle et/ou les vitesses de déclenchement cumulatives d'au moins une zone de commande du tronc cérébral.

4. Système de neuromodulation (10, 110) selon la revendication 1, **caractérisé en ce que** l'au moins un module d'entrée de signal (12, 112) comprend un module de commutation d'entrée, dans lequel le module de commutation d'entrée est configuré pour commuter entre des signaux indiquant une pression artérielle et des vitesses de déclenchement cumulatives d'au moins une zone de commande du tronc cérébral.

5. Système de neuromodulation (10, 110) selon l'une des revendications précédentes, **caractérisé en ce que** le module de commande (14, 114) comprend un module d'anticipation, dans lequel le module d'anticipation est configuré et agencé pour prendre en compte au moins un effet prédictif de la stimulation pour ajuster le coefficient β avec une fenêtre temporelle spécifiée.

6. Système de neuromodulation (10, 110) selon l'une des revendications précédentes, **caractérisé en ce que** le module de commande (14, 114) est configuré pour comprendre des paramètres de commande de paradigme de stimulation.

7. Système de neuromodulation (210) selon l'une des revendications précédentes, **caractérisé en ce que**
le module d'entrée de signaux est ou comprend au moins un élément de détection (212) configuré pour détecter un signal indiquant un paramètre physiologique d'un patient, au moins un module de cartographie spatiale (216) configuré pour relier des configurations (Config) spatiales de stimulation par électrode ciblant les fibres afférentes dans les racines dorsales/postérieures à au moins un effet physiologique,
au moins un module de cartographie de paramètres (218) configuré pour préparer des paramètres de stimulation pour le module de commande (214) sur la base de données reçues de l'élément de détection (212) et/ou du module de cartographie spatiale (216).

8. Système de neuromodulation (10) selon la revendication 7,
**caractérisé en ce que** le paramètre physiologique est au moins l'un parmi oxygénation, pression artérielle du patient, pression de perfusion de la moelle épinière du patient, posture du patient et/ou position du patient.

9. Système (10) selon la revendication 7, **caractérisé en ce que** le système comprend au moins un élément de stimulation comprenant au moins un réseau d'électrodes A comprenant plusieurs électrodes E.

10. Système (10) selon la revendication 9, **caractérisé en ce que** le système comprend au moins un module de cartographie temporale configuré pour relier des configurations temporales de stimulation par électrodes à au moins un effet physiologique.

11. Système (10) selon l'une des revendications 9 à 10,
**caractérisé en ce que** le module de commande (14) est configuré pour identifier une valeur cible pour une fonction autonome sur la base d'un signal fourni par l'élément de détection (12).

12. Système de neuromodulation (10) selon l'une des revendications 9 à 11, **caractérisé en ce que** le module de cartographie spatiale (16) isole des électrodes E clés en fonction d'une localisation anatomique des fibres afférentes dans les racines dorsales et d'une procédure d'apprentissage initiée au niveau de ces électrodes E pour optimiser la configuration des électrodes E environnantes.

13. Système de neuromodulation (10) selon l'une des revendications précédentes, **caractérisé en ce que** les paramètres de stimulation comprennent au moins une fréquence, une amplitude et une largeur d'impulsion, dans lequel la fréquence est de 10 Hz à 10 kHz, l'amplitude de 0 à 1 A ou 0 à 15 V et la largeur d'impulsion de 1 à 500 µs.

14. Système de neuromodulation (10) selon l'une des revendications précédentes, **caractérisé en ce que** le module de cartographie spatiale (16) est configuré pour effectuer une procédure d'apprentissage par renforcement, dans lequel la procédure d'apprentissage par renforcement fait partie du processus visant à relier des configurations (Config) spatiales de stimulation par électrodes ciblant les fibres afférentes dans les racines dorsales/postérieures à au moins un effet physiologique.

15. Système de neuromodulation (10) selon l'une des revendications précédentes, **caractérisé en ce que** le module de cartographie spatiale (16) est configuré pour effectuer une phase de cartographie spatiale pour identifier une configuration (Config) d'électrodes appropriée en termes d'électrodes E sélectionnées et leur agencement spatial dans une première étape et une phase de cartographie de paramètres pour ajuster des paramètres de stimulation pour la stimulation fournie par les électrodes E sélectionnées dans la première étape.
